# EUROPEAN PATENT APPLICATION

(11) **EP 4 483 883 A1**
(43) Date of publication of application: **01.01.2025**
(21) Application number: 23773794.5
(22) Date of filing: 20.03.2023
(51) Int. Cl.: A61K 31/519, A61P 35/00

(54) **USE OF PYRROLOPYRIMIDINE COMPOUND IN TREATMENT OF MEDIUM-RISK OR HIGH-RISK MYELOFIBROSIS**

(30) Priority: 21.03.2022 CN 202210283950
(71) Applicant: Chia Tai Tianqing Pharmaceutical Group Co., Ltd, Lianyungang, Jiangsu 222062 (CN)
(72) Inventor: DING, Dawei, Lianyungang, Jiangsu 222062 (CN); YU, Ding, Lianyungang, Jiangsu 222062 (CN); WANG, Xunqiang, Lianyungang, Jiangsu 222062 (CN); LAO, Shanli, Lianyungang, Jiangsu 222062 (CN)
(74) Representative: Müller Schupfner & Partner Patent- und Rechtsanwaltspartnerschaft mbB (Muc)
(86) International application number: PCT/CN2023/082533
(87) International publication number: WO 2023/179547

(57) **Abstract**

The present invention belongs to the field of medicinal chemistry, relates to use of a pyrrolopyrimidine compound in the treatment of medium-risk or high-risk myelofibrosis, and specifically relates to use of a compound of formula I, a stereoisomer thereof, a pharmaceutically acceptable salt thereof, or a pharmaceutical composition comprising same in the treatment of medium-risk or high-risk myelofibrosis. The compound of formula **I,** the stereoisomer thereof, the pharmaceutically acceptable salt thereof, or the pharmaceutical composition thereof has a good therapeutic effect.

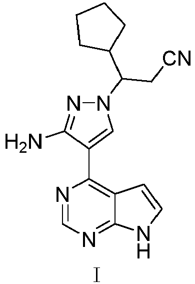

## Description

### CROSS-REFERENCE TO RELATED APPLICATIONS

The present application claims priority and benefit to the Chinese Patent Application No. 202210283950.4 filed with National Intellectual Property Administration, PRC on Mar. 21, 2022, the disclosure of which is incorporated herein by reference in its entirety.

### TECHNICAL FIELD

The present application belongs to the field of medicinal chemistry, relates to use of a pyrrolopyrimidine compound for treating medium-risk or high-risk myelofibrosis, and particularly relates to use of a compound of formula I, a stereoisomer thereof, or a pharmaceutically acceptable salt thereof for treating medium-risk or high-risk myelofibrosis.

### BACKGROUND

Janus kinase (JAK) is a group of non-receptor tyrosine kinases (PTKs), which exists in cells and transmits cytokine stimulation signals through the JAK-STAT pathway. The JAK-STAT pathway transmits extracellular chemical signals through the cell membrane to the gene promoter of DNA in the nucleus, which ultimately causes changes in DNA transcription and activity. The JAK-STAT pathway consists of three major components: 1) a receptor; 2) the JAK; and 3) a signal transducer and activator of transcription (STAT) protein. The receptor can be activated by interferons, interleukins, growth factors, or other chemical messengers, leading to the autophosphorylation of JAK; the STAT protein binds to the phosphorylated receptor, such that the STAT protein is phosphorylated by JAK; then the phosphorylated STAT protein is separated from the receptor, dimerized and translocated into the nucleus to bind to the specific sites on DNA and alter the transcription (Scott, M. J., C. J. Godshall, et al., (2002) "Jaks, STATs, Cytokines, and Sepsis", Clin Diagn Lab Immunol 9(6): 1153-9).

The JAK family plays a role in cell proliferation and functional cytokine-dependent regulation of immune responses. Currently, there are four known mammalian JAK family members: JAK1, JAK2, JAK3, and TYK2 (tyrosine kinase 2). The size of JAK proteins ranges from 120-140 kDa. A JAK protein contains 7 conserved JAK homology (JH) domains, one of which is a functional catalytic kinase domain, and another of which is a pseudokinase domain that effectively exerts a regulatory function and/or acts as a docking site for STAT proteins (Scott, Godshall, et al., 2002, supra).

Myelofibrosis (MF) is a chronic, progressive myeloproliferative neoplasm caused by collagen proliferation in hematopoietic tissues of the bone marrow, which results in the generation of fibrous tissues that severely affect hematopoietic function. Myelofibrosis includes primary myelofibrosis (PMF), post-polycythemia vera myelofibrosis (post-PV-MF), and post-essential thrombocythemia myelofibrosis (post-ET-MF), which are mainly characterized by various degrees of bone marrow fibrous tissue proliferation, blood cell abnormalities/anemia, splenomegaly, and systemic symptoms (e.g., weakness, night sweats, weight loss, upper left abdominal distention, and the like.). MF generally progresses slowly. Patients in the early stage generally survive for 10 or more years, but the overall median survival time of patients is 5-7 years, with a median survival time of 2.7 years for medium-risk or high-risk myelofibrosis patients.

Currently, a safe and effective treatment method for MF is lacking. For patients in the early to middle stages, where the survival is generally longer, the common treatment strategy is symptomatic treatment, i.e., giving appropriate treatment according to the presence or absence of clinical characteristics of anemia, splenomegaly, constitutional symptoms, symptomatic extramedullary hematopoiesis, and the like in the patients, and in conjunction with prognostic analysis.

### BRIEF SUMMARY

In one aspect, the present application provides a compound of formula I, a stereoisomer thereof, or a pharmaceutically acceptable salt thereof for use in treating medium-risk or high-risk myelofibrosis with a previous failure of treatment with ruxolitinib:

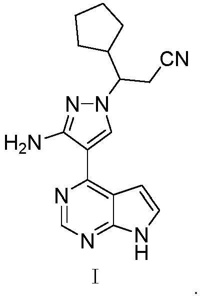

In another aspect, the present application provides a pharmaceutical composition for use in treating medium-risk or high-risk myelofibrosis with a previous failure of treatment with ruxolitinib, which comprises a compound of formula **I,** a stereoisomer thereof, or a pharmaceutically acceptable salt thereof.

In another aspect, the present application provides a method for treating medium-risk or high-risk myelofibrosis with a previous failure of treatment with ruxolitinib, which comprises administering to a patient an effective amount of the compound of formula **I,** the stereoisomer thereof, or the pharmaceutically acceptable salt thereof, or the pharmaceutical composition thereof as described above.

In another aspect, the present application provides use of the compound of formula **I,** the stereoisomer thereof, or the pharmaceutically acceptable salt thereof, or the pharmaceutical composition thereof as described above for preparing a medicament for treating medium-risk or high-risk myelofibrosis with a previous failure of treatment with ruxolitinib.

In another aspect, the present application provides use of the compound of formula **I,** the stereoisomer thereof, or the pharmaceutically acceptable salt thereof, or the pharmaceutical composition thereof as described above for treating medium-risk or high-risk myelofibrosis with a previous failure of treatment with ruxolitinib.

### SUMMARY

The present application provides a compound of formula **I,** a stereoisomer thereof, or a pharmaceutically acceptable salt for use in treating medium-risk or high-risk myelofibrosis with a previous failure of treatment with ruxolitinib:

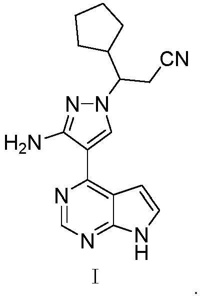

In another aspect, the present application provides a pharmaceutical composition for use in treating medium-risk or high-risk myelofibrosis with a previous failure of treatment with ruxolitinib, which comprises a compound of formula I, a stereoisomer thereof, or a pharmaceutically acceptable salt thereof.

In another aspect, the present application provides a method for treating medium-risk or high-risk myelofibrosis with a previous failure of treatment with ruxolitinib, which comprises administering to a patient in need thereof an effective amount of the compound of formula **I,** the stereoisomer thereof, or the pharmaceutically acceptable salt thereof, or the pharmaceutical composition comprising the compound of formula **I,** the stereoisomer thereof, or the pharmaceutically acceptable salt thereof as described above.

In another aspect, the present application provides use of the compound of formula **I,** the stereoisomer thereof, or the pharmaceutically acceptable salt thereof, or the pharmaceutical composition comprising the compound of formula **I,** the stereoisomer thereof, or the pharmaceutically acceptable salt thereof as described above for preparing a medicament for treating medium-risk or high-risk myelofibrosis with a previous failure of treatment with ruxolitinib.

In another aspect, the present application provides use of the compound of formula **I,** the stereoisomer thereof, or the pharmaceutically acceptable salt thereof, or the pharmaceutical composition comprising the compound of formula **I,** the stereoisomer thereof, or the pharmaceutically acceptable salt thereof as described above for treating medium-risk or high-risk myelofibrosis with a previous failure of treatment with ruxolitinib.

In some embodiments of the present application, the compound of formula **I,** the stereoisomer thereof, or the pharmaceutically acceptable salt thereof described herein is used as a single active agent. In some embodiments of the present application, the compound of formula **I,** the stereoisomer thereof, or the pharmaceutically acceptable salt thereof described herein is used as the sole active agent.

In some embodiments of the present application, the compound of formula **I,** the stereoisomer thereof, or the pharmaceutically acceptable salt thereof described herein may be a pharmaceutical composition comprising a therapeutically effective amount of the compound of formula **I,** the stereoisomer thereof, or the pharmaceutically acceptable salt thereof.

In some embodiments of the present application, the pharmaceutical composition described herein may further comprise a pharmaceutically acceptable excipient (also referred to as "pharmaceutically acceptable carrier", or "pharmaceutic excipient"). The pharmaceutical composition of the present application may be prepared by combining the compound of the present application with a suitable pharmaceutically acceptable excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol. The pharmaceutical composition of the present application may be manufactured using methods well known in the art, such as conventional mixing, dissolving, granulating, dragee-making, levigating, emulsifying, and lyophilizing. Suitable excipients include, but are not limited to: binders, diluents, wetting agents, disintegrants, lubricants, glidants, sweeteners, flavoring agents, or the like.

In some embodiments of the present application, the pharmaceutical composition is a formulation suitable for oral administration, including tablets, capsules, powders, granules, dripping pills, pastes, pulvis, and the like, preferably tablets and capsules. The oral formulation may be prepared by a conventional method using a pharmaceutically acceptable carrier well known in the art. The pharmaceutically acceptable carrier includes diluents, binders, wetting agents, disintegrants, lubricants, and the like. The diluents include microcrystalline cellulose, mannitol, lactose, sucrose, starch, pregelatinized starch, dextrin, and the like, or a mixture thereof; the binders include hydroxypropyl methylcellulose, carboxymethyl cellulose, carboxymethylcellulose sodium, ethyl cellulose, methyl cellulose, hydroxypropyl cellulose, low-substituted hydroxypropyl cellulose, gelatin, polyvinylpyrrolidone, starch, sucrose, glucose, and the like, or a mixture thereof; the wetting agents include magnesium stearate, talcum powder, polyethylene glycol, sodium dodecyl sulfate, silica gel micropowder, and the like, or a mixture thereof; the disintegrants include sodium carboxymethyl starch, dry starch, microcrystalline cellulose, hydroxyethyl methylcellulose, carboxymethylcellulose sodium, carboxymethylcellulose calcium, croscarmellose sodium, low-substituted hydroxypropyl methylcellulose, crospovidone, and the like, or a mixture thereof; and the lubricants include magnesium stearate, colloidal silicon dioxide, talcum powder, polyethylene glycol, stearic acid, sodium stearyl fumarate, and the like, or a mixture thereof. The pharmaceutical excipients also include coloring agents, sweeteners, coating agents, and the like.

### Compound of Formula I, Stereoisomer Thereof, or Pharmaceutically Acceptable Salt Thereof

In some embodiments of the present application, the compound of formula I described herein is a compound of formula II

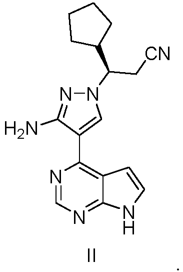

The compound of formula I and the compound of formula II of the present application may be prepared with reference to the preparation methods in Patent Nos. WO2016095805 and WO2017215627.

In some embodiments of the present application, the compound of formula **I** or the compound of formula II is administered in the form of a free base. In some embodiments of the present application, the compound of formula **I** or the compound of formula II is administered in the form of a crystalline free base. In some embodiments of the present application, the crystalline free base of the compound of formula II may be selected from the group consisting of crystal form A and crystal form B disclosed in Patent No. WO2017215630.

In some embodiments of the present application, the compound of formula II is administered in the form of a hydrochloride salt. In some embodiments of the present application, the hydrochloride salt of the compound of formula II may be selected from the group consisting of hydrochloride salts disclosed in Patent No. WO2017101777.

### Myelofibrosis

In some embodiments of the present application, the myelofibrosis includes primary myelofibrosis (PMF), post-polycythemia vera myelofibrosis (Post-PV-MF), and post-essential thrombocythemia myelofibrosis (Post-ET-MF).

In some embodiments of the present application, the failure of treatment comprises being refractory and/or being recurrent and/or being intolerant.

In some embodiments of the present application, being refractory is that a patient has been treated with ruxolitinib for a cumulative total of ≥ 3 months, and the spleen volume is reduced by < 10% or increased as detected by an MRI or CT scan compared with the spleen volume before taking ruxolitinib, or the spleen volume is reduced by < 30% or increased as assessed by palpation compared with the spleen volume before taking a JAK inhibitor (e.g., ruxolitinib).

In some embodiments of the present application, being recurrent is that a patient has been treated with ruxolitinib for a cumulative total of ≥ 3 months, the spleen enlarges again after a response compared with the spleen volume before taking a JAK inhibitor (e.g., ruxolitinib), and the spleen volume is reduced by < 10% as detected by an MRI or CT scan, or is reduced by < 30% as assessed by palpation compared with the spleen volume before taking ruxolitinib.

In some embodiments of the present application, being intolerant is that a patient has been treated with ruxolitinib for a cumulative total of ≥ 28 days (except for allergy), and the patient still requires red cell transfusions or has an increase in the frequency of transfusions during treatment with ruxolitinib, or the patient experiences a reduction in platelet count of grade 3 or higher, or anemia of grade 3 or higher, or hematoma or hemorrhage of grade 3 or higher.

In some embodiments of the present application, the myelofibrosis is medium-risk or high-risk myelofibrosis that is refractory and/or recurrent and/or intolerant to ruxolitinib.

In some embodiments of the present application, the myelofibrosis is myelofibrosis with which a patient has been treated with ruxolitinib for a cumulative total of ≥ 3 months, and the spleen volume is reduced by < 10% or increased as detected by an MRI or CT scan compared with the spleen volume before taking ruxolitinib, or the spleen volume is reduced by < 30% or increased as assessed by palpation compared with the spleen volume before taking ruxolitinib.

In some embodiments of the present application, the myelofibrosis is myelofibrosis with which a patient has been treated with ruxolitinib for a cumulative total of ≥ 3 months, the spleen enlarges again after a response compared with the spleen volume before taking ruxolitinib, and the spleen volume is reduced by < 10% as detected by an MRI or CT scan, or is reduced by < 30% as assessed by palpation compared with the spleen volume before taking ruxolitinib.

In some embodiments of the present application, the myelofibrosis is myelofibrosis with which a patient has been treated with ruxolitinib for a cumulative total of ≥ 28 days (except for allergy) and the patient still requires red cell transfusions or has an increase in the frequency of transfusions during treatment with ruxolitinib, or the patient experiences a reduction in platelet count of grade 3 or higher, or anemia of grade 3 or higher, or hematoma or hemorrhage of grade 3 or higher.

In some embodiments of the present application, the myelofibrosis is that a splenic margin of a patient reaches or extends at least 5 cm below the costal margin as assessed by palpation.

In some embodiments of the present application, the myelofibrosis is that both peripheral blood blast cells and bone marrow blast cells in the patient are ≤ 10%.

In some embodiments of the present application, the patient with myelofibrosis does not receive growth factors and/or colony stimulating factors and/or thrombopoietic factors and/or platelet infusions within 2 weeks before the examination, and hemoglobin (HGB) is ≥ 80 g/L, platelet count (PLT) is ≥ 100 × 10⁹/L, and absolute value of neutrophil count (NEUT) is ≥ 1.0 × 10⁹/L within 7 days before the first administration.

In the present application, the myelofibrosis includes myelofibrosis with a mutation in a gene including but not limited to, JAK2 (e.g., JAK2 V617F), MPL, CALR, ASXL1/SRSF2/IDH1/21, JAK2 exon 12, TP53, SH2B3/IDH2/U2AF1/SF3B1/EZH2/TP53, MPL p.W515L/K, CALR Type 1/Type 1-like, a triple-negative gene (no mutations in JAK2, MPL, and CALR), ASXL1, EZH2, IDH1/2, SRSF2, SF3B1, CALR/ASXL1, TP53, or U2AF1 Q157.

### Administration Regimen

In some embodiments of the present application, the administration cycle for treating medium-risk or high-risk myelofibrosis in a patient is 2-6 weeks. In some embodiments of the present application, the administration cycle for treating medium-risk or high-risk myelofibrosis in a patient is 2 weeks, 3 weeks, 4 weeks, 5 weeks, 6 weeks, or a range formed by any of the aforementioned values. In some embodiments of the present application, the administration cycle for treating medium-risk or high-risk myelofibrosis in a patient is 4 weeks. In some embodiments of the present application, the compound of formula I, the stereoisomer thereof, or the pharmaceutically acceptable salt thereof, or the pharmaceutical composition comprising the same of the present application is administered to a patient with medium-risk or high-risk myelofibrosis with a failure of treatment with ruxolitinib at an administration cycle of 2-6 weeks, for example, 3-5 weeks, 2 weeks, 3 weeks, 4 weeks, 5 weeks, or 6 weeks. In some embodiments of the present application, the compound of formula I, the stereoisomer thereof, or the pharmaceutically acceptable salt thereof, or a pharmaceutical composition comprising the same of the present application is administered to the patient daily or at regular intervals during the administration cycle. The amount of the compound, the stereoisomer thereof, or the pharmaceutically acceptable salt thereof of the present application may be determined on the basis of the severity of the disease, the response to the disease, any treatment-related toxicity, and the age and health condition of the patient. For example, it may be determined on the basis of the subject/patient's blood routine examination results, which include platelet count, neutrophil count, or hemoglobin concentration, and the like. In some embodiments, a daily dose of the compound, the stereoisomer thereof, or the pharmaceutically acceptable salt thereof of the present application administered is 1 mg to 100 mg. In some embodiments, the daily dose of the compound, the stereoisomer thereof, or the pharmaceutically acceptable salt thereof of the present application administered may be selected from the group consisting of 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or 100 mg, or a range of any two of the foregoing values as endpoints or any value therein, for example, 1 mg to 90 mg, 5 mg to 80 mg, 10 mg to 70 mg, 15 mg to 60 mg, 20 mg to 50 mg, 20 mg to 40 mg, 30 mg to 40 mg, and the like. In some specific embodiments, the daily dose of the compound, the stereoisomer thereof, or the pharmaceutically acceptable salt thereof of the present application administered may be selected from the group consisting of 1 mg to 50 mg, 5 mg to 50 mg, 5 mg to 45 mg 5 mg to 40 mg, 10 mg to 35 mg, 10 mg to 30 mg, 20 mg to 40 mg, and 30 mg to 40 mg. In some specific embodiments, the daily dose of the compound, the stereoisomer thereof, or the pharmaceutically acceptable salt thereof of the present application administered may be selected from the group consisting of 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value therein, for example, 2 mg to 50 mg, 10 mg to 40 mg, 5 mg to 30 mg, 5 mg to 20 mg, 20 mg to 40 mg, 30 mg to 40 mg, and the like.

The compound, the stereoisomer thereof, or the pharmaceutically acceptable salt thereof of the present application may be administered once or multiple times daily. In some embodiments, the compound, the stereoisomer thereof, or the pharmaceutically acceptable salt thereof of the present application is administered once or twice daily. In some embodiments, the compound, the stereoisomer thereof, or the pharmaceutically acceptable salt thereof of the present application is administered twice daily.

The compound, the stereoisomer thereof, or the pharmaceutically acceptable salt thereof of the present application may also be administered in a single dose. In one embodiment, the compound, the stereoisomer thereof, or the pharmaceutically acceptable salt thereof of the present application is administered in a single dose once or twice daily. In one embodiment, the compound, the stereoisomer thereof, or the pharmaceutically acceptable salt thereof of the present application is administered in the form of a single-dose oral solid formulation once or twice daily. In one specific embodiment, the compound, the stereoisomer thereof, or the pharmaceutically acceptable salt thereof of the present application is administered in the form of a single-dose oral solid formulation twice daily.

The compound, the stereoisomer thereof, or the pharmaceutically acceptable salt thereof of the present application may also be administered in multiple doses. In one embodiment, the compound, the stereoisomer thereof, or the pharmaceutically acceptable salt thereof of the present application is administered in multiple doses once or twice daily. In one embodiment, the compound, the stereoisomer thereof, or the pharmaceutically acceptable salt thereof of the present application is administered in the form of a multiple-dose oral solid formulation once or twice daily. In one specific embodiment, the compound, the stereoisomer thereof, or the pharmaceutically acceptable salt thereof of the present application is administered in the form of a multiple-dose oral solid formulation twice daily. In some embodiments of the present application, the compound of formula I, the stereoisomer thereof, or the pharmaceutically acceptable salt thereof is provided in the form of a pharmaceutical composition, preferably a single-dose pharmaceutical composition. In some embodiments, the pharmaceutical composition comprises 1 mg to 50 mg of the compound, the stereoisomer thereof, or the pharmaceutically acceptable salt thereof of the present application. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value therein of the compound, the stereoisomer thereof, or the pharmaceutically acceptable salt thereof of the present application, for example, 2 mg to 50 mg, 10 mg to 40 mg, 5 mg to 30 mg, 5 mg to 20 mg, 20 mg to 40 mg, 30 mg to 40 mg, and the like.

In some embodiments of the present application, the pharmaceutical composition of the compound of formula I, the stereoisomer thereof, or the pharmaceutically acceptable salt thereof is provided in a daily dose. In some embodiments of the present application, the pharmaceutical composition of the compound of formula I, the stereoisomer thereof, or the pharmaceutically acceptable salt thereof is administered in twice-daily doses. In some embodiments of the present application, the twice-daily doses are in the same dose.

In some embodiments of the present application, the pharmaceutical composition of the compound of formula I, the stereoisomer thereof, or the pharmaceutically acceptable salt thereof is administered in twice-daily doses, with each dose being in a single dose or multiple doses.

In some embodiments of the present application, the pharmaceutical composition of the compound of formula I, the stereoisomer thereof, or the pharmaceutically acceptable salt thereof is administered in twice-daily doses, with each dose being in multiple doses consisting of single doses of 5 mg, 10 mg, 15 mg, and/or 20 mg of the compound of formula **I,** the stereoisomer thereof, or the pharmaceutically acceptable salt thereof. In some embodiments of the present application, the pharmaceutical composition consists of single doses of 5 mg of the compound of formula **I,** the stereoisomer thereof, or the pharmaceutically acceptable salt thereof.

In some embodiments of the present application, the pharmaceutical composition of the compound of formula **I,** the stereoisomer thereof, or the pharmaceutically acceptable salt thereof is packaged in a kit. The kit also comprises instructions on the compound of formula **I,** the stereoisomer thereof, or the pharmaceutically acceptable salt thereof for use in treating medium-risk or high-risk myelofibrosis following previous treatment with ruxolitinib; further preferably, the kit also comprises instructions on the compound of formula **I,** the stereoisomer thereof, or the pharmaceutically acceptable salt thereof for use in treating medium-risk or high-risk myelofibrosis that is refractory and/or recurrent and/or intolerant following previous treatment with ruxolitinib.

In some embodiments of the present application, in the method or use for treating medium-risk or high-risk myelofibrosis that has previously treatment with ruxolitinib, the pharmaceutical composition of the compound of formula **I,** the stereoisomer thereof, or the pharmaceutically acceptable salt thereof is provided in a form of a daily dose and is administered as follows: the pharmaceutical composition of the compound of formula **I,** the stereoisomer thereof, or the pharmaceutically acceptable salt thereof is administered once or twice daily; in some embodiments of the present application, the pharmaceutical composition of the compound of formula **I,** the stereoisomer thereof, or the pharmaceutically acceptable salt thereof is administered twice daily in the same dose; in some embodiments of the present application, the pharmaceutical composition of the compound of formula **I,** the stereoisomer thereof, or the pharmaceutically acceptable salt thereof is administered twice daily in the same dose at an interval of at least 8 h; in some embodiments of the present application, the pharmaceutical composition of the compound of formula **I,** the stereoisomer thereof, or the pharmaceutically acceptable salt thereof is administered twice daily in the same dose at an interval of 12 h.

In some embodiments of the present application, in the method or use for treating medium-risk or high-risk myelofibrosis following previous treatment with ruxolitinib, the pharmaceutical composition of the compound of formula **I,** the stereoisomer thereof, or the pharmaceutically acceptable salt thereof is provided in a form of a daily dose and is administered as follows: the pharmaceutical composition of the compound of formula **I,** the stereoisomer thereof, or the pharmaceutically acceptable salt thereof is administered twice daily on an empty stomach in the same dose at an interval of 12 h.

In some embodiments of the present application, in the method or use for treating medium-risk or high-risk myelofibrosis following previous treatment with ruxolitinib, each dose of the pharmaceutical composition of the compound of formula **I,** the stereoisomer thereof, or the pharmaceutically acceptable salt thereof is 1 mg to 50 mg. In some embodiments, each dose of the pharmaceutical composition is 5 mg to 30 mg. In some embodiments, each dose of the pharmaceutical composition is 5 mg, 10 mg, 15 mg, 20 mg, 25 mg, 30 mg, or a range of any two of the foregoing values as endpoints or any value therein, for example, 5 mg to 30 mg, 5 mg to 25 mg, 5 mg to 20 mg, 5 mg to 15 mg, 10 mg to 20 mg, 10 mg to 20 mg, 10 mg to 15 mg, and the like. In some embodiments, each dose of the pharmaceutical composition is 5 mg, 10 mg, 15 mg, 20 mg, 25 mg, or 30 mg. In some embodiments, each dose of the pharmaceutical composition is 15 mg.

In some embodiments of the present application, in the method or use for treating medium-risk or high-risk myelofibrosis following previous treatment with ruxolitinib, a daily dose of the pharmaceutical composition of the compound of formula **I,** the stereoisomer thereof, or the pharmaceutically acceptable salt thereof is 1 mg to 50 mg. In some embodiments, the daily dose of the pharmaceutical composition is 5 mg to 50 mg. In some embodiments, the daily dose of the pharmaceutical composition is 5 mg, 10 mg, 15 mg, 20 mg, 25 mg, 30 mg, 35 mg, 40 mg, 45 mg, 50 mg, or a range of any two of the foregoing values as endpoints or any value therein, for example, 5 mg to 50 mg, 10 mg to 50 mg, 15 mg to 40 mg, 15 mg to 30 mg, 20 mg to 40 mg, 20 mg to 30 mg, and the like. In some embodiments, the daily dose of the pharmaceutical composition is 15 mg, 20 mg, 25 mg, 30 mg, 35 mg, 40 mg, 45 mg, or 50 mg. In some embodiments, the daily dose of the pharmaceutical composition is 30 mg.

In some embodiments of the present application, 28 days constitute one treatment cycle, in which the pharmaceutical composition of the compound of formula **I,** the stereoisomer thereof, or the pharmaceutically acceptable salt thereof is administered daily consecutively from day 1 to day 28.

In some embodiments of the present application, 28 days constitute one treatment cycle, in which the pharmaceutical composition of the compound of formula **I,** the stereoisomer thereof, or the pharmaceutically acceptable salt thereof is administered twice daily consecutively from day 1 to day 28.

In some embodiments of the present application, 28 days constitute one treatment cycle, in which the pharmaceutical composition of the compound of formula **I,** the stereoisomer thereof, or the pharmaceutically acceptable salt thereof is administered twice daily consecutively from day 1 to day 28, and each dose of the pharmaceutical composition is 15 mg.

In some embodiments of the present application, 28 days constitute one treatment cycle, in which the pharmaceutical composition of the compound of formula **I,** the stereoisomer thereof, or the pharmaceutically acceptable salt thereof is administered twice daily consecutively from day 1 to day 28, each dose of the pharmaceutical composition is 15 mg, and the daily dose of the pharmaceutical composition is 30 mg.

In some embodiments of the present application, 28 days constitute one treatment cycle, in which a total dose of 140-840 mg of the pharmaceutical composition of the compound of formula **I,** the stereoisomer thereof, or the pharmaceutically acceptable salt thereof (calculated based on the active ingredient, the compound of formula (I)) is administered. In some embodiments of the present application, the total dose of the pharmaceutical composition of the compound of formula **I,** the stereoisomer thereof, or the pharmaceutically acceptable salt thereof is selected from the group consisting of 140 mg, 280 mg, 420 mg, 560 mg, 700 mg, 840 mg, and a range formed by any two of the foregoing values (calculated based on the active ingredient, the compound of formula (I)). In some embodiments of the present application, the total dose of the pharmaceutical composition of the compound of formula **I,** the stereoisomer thereof, or the pharmaceutically acceptable salt thereof is preferably 560-840 mg (calculated based on the active ingredient, the compound of formula (I)). In some embodiments of the present application, the total dose of the pharmaceutical composition of the compound of formula **I,** the stereoisomer thereof, or the pharmaceutically acceptable salt thereof is preferably 840 mg (calculated based on the active ingredient, the compound of formula (I)).

In some embodiments of the present application, the treatment cycle is repeated as long as the disease is still under control and the administration regimen is clinically tolerable.

The compound, the stereoisomer thereof, or the pharmaceutically acceptable salt thereof of the present application may be administered by various routes, including but not limited to: oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations. In one specific embodiment, the route is oral administration.

In some embodiments of the present application, the pharmaceutical composition of the compound of formula **I,** the stereoisomer thereof, or the pharmaceutically acceptable salt thereof may be formulated in a form suitable for oral administration to a human, for example, including but not limited to, tablet, pill, capsule, powder, or granule, and the like.

In some embodiments of the present application, the pharmaceutical composition of the compound of formula **I,** the stereoisomer thereof, or the pharmaceutically acceptable salt thereof may be in the form of a single-day or single-cycle administration formulation. In some embodiments, the pharmaceutical composition of the compound of formula **I,** the stereoisomer thereof, or the pharmaceutically acceptable salt thereof may be in the form of a single-day administration formulation comprising 1 or more (e.g., 1-6) discrete units, with each of the discrete units comprising 5-30 mg of the compound of formula **I.** In some embodiments, the pharmaceutical composition of the compound of formula **I,** the stereoisomer thereof, or the pharmaceutically acceptable salt thereof may be in the form of a single-cycle administration formulation comprising discrete units, with each of the discrete units comprising 5-30 mg of the compound of formula **I.**

In some embodiments of the present application, the pharmaceutical composition of the compound of formula **I,** the stereoisomer thereof, or the pharmaceutically acceptable salt thereof is in the form of an oral tablet (e.g., scored tablet).

In some embodiments of the present application, a single dose of the oral tablet of the compound of formula **I,** the stereoisomer thereof, or the pharmaceutically acceptable salt thereof is 5 mg.

### Technical Effects

The compound of formula **I,** the stereoisomer thereof, or the pharmaceutically acceptable salt thereof, or the pharmaceutical composition thereof of the present application may have good therapeutic effects, including but not limited to, better disease control rate, longer survival (e.g., median survival, progression-free survival or overall survival) and longer duration of response (DOR) to the treatment of the disease. The compound of formula **I,** the stereoisomer thereof, or the pharmaceutically acceptable salt thereof, or the pharmaceutical composition thereof of the present application may also have good safety, including but not limited to, a lower incidence of adverse events, while having good therapeutic effects.

### Definitions and Explanations

Unless otherwise stated, the terms used herein shall have the following meanings. A certain term, unless otherwise specifically defined, should not be considered uncertain or unclear, but construed according to its common meaning in the art. When referring to a trade name, it is intended to refer to its corresponding commercial product or its active ingredient.

As used herein, unless otherwise stated, the terms "contain", "contains", "containing", "include", "includes", "including", "comprise", "comprises", "comprising", or equivalents thereof are open-ended statements and mean that elements, components, and steps that are not specified may be encompassed in addition to those listed.

All patents, patent applications, and other identified publications are explicitly incorporated herein by reference for the purpose of description and disclosure. Any reference to these publications herein shall not be construed as an admission that the publications form part of the commonly recognized knowledge in the art.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases. The pharmaceutically acceptable salt described herein is selected from the group consisting of a maleate salt, a hydrochloride salt, a hydrobromide salt, a sulfate salt, a phosphate salt, a nitrate salt, an acetate salt, a lactate salt, a malonate salt, a succinate salt, a fumarate salt, a malate salt, a mandelate salt, a tartrate salt, a citrate salt, an ascorbate salt, a palmitate salt, a benzoate salt, a phenylacetate salt, a cinnamate salt, a salicylate salt, a methanesulfonate salt, a benzenesulfonate salt, or a methylbenzenesulfonate salt.

As used herein, the amount of the compound of formula I, for example, the amount administered, the dose, or the amount in the pharmaceutical composition, is calculated based on its free base form.

As used herein, if the compound in the pharmaceutical combination of the present application has, for example, at least one basic site, it may form an acid addition salt. If needed, it may further form a corresponding acid addition salt with additionally present basic sites. A compound with at least one acidic group (e.g., -COOH) may further form a salt with a base. If the compound comprises, for example, both carboxyl and amino, it may further form an inner salt.

The compound of the present application may be asymmetrical, for example, having one or more stereoisomers. Unless otherwise stated, all stereoisomers are included, for example, enantiomers and diastereoisomers. The compound of the present application containing asymmetrical carbon atoms may be separated in an optically pure form or in a racemic form. The optically pure form may be separated from a racemic mixture or may be synthesized using a chiral raw material or a chiral reagent.

The term "patient" is a mammal, such as a human, dog, cow, horse, pig, sheep, goat, cat, mouse, rabbit, rat, or transgenic non-human animal. In some embodiments, the patient is a human.

The term "pharmaceutical composition" refers to a mixture consisting of one or more of the compounds or the pharmaceutical combinations thereof or the salts or stereoisomers thereof of the present application and a pharmaceutically acceptable excipient. The pharmaceutical composition is intended to facilitate the administration of the compound or the pharmaceutical combination thereof of the present application to a subject.

The term "treatment" generally refers to obtaining desired pharmacological and/or physiological effects, including partially or completely stabilizing or curing a disease and/or an effect that the disease has. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application for (i) treating a specific disease, condition, or disorder, or (ii) alleviating, ameliorating, or eliminating one or more symptoms of a specific disease, condition, or disorder. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "single dose" refers to the smallest unit of packaging containing a certain quantity of pharmaceutical product; for example, in a box of seven capsules, each capsule is a single dose; or a vial of injection is a single dose.

The term "multiple doses" consists of multiple single doses.

The terms "administer", "administration" and "administering" refer to physically introducing the composition comprising a therapeutic agent to an individual using any of a variety of methods and delivery systems known to those skilled in the art. In certain embodiments, the administration is oral administration.

The term "daily dose" refers to a dose administered to a patient per day.

The term "each dose" refers to a dose administered to a patient in a single administration.

The terms "day", "daily", and the like, when referred to in an administration regimen, refer to the time within a calendar day that starts at midnight and ends at the next midnight.

Unless otherwise specified clearly in the context, singular terms herein encompass plural referents, and vice versa. Similarly, unless otherwise specified clearly in the context, the word "or" herein is intended to include "and".

### DETAILED DESCRIPTION

The present invention is illustrated in more details through specific examples. The following examples are provided for illustrative purposes only and are not intended to limit the present invention in any way.

### Preparation Example 1: Preparation of Solid Pharmaceutical Composition in 5 mg Tablets

The formula for a solid pharmaceutical composition in 5 mg tablets is shown in Table 1:

**Table 1 Formula for 5 mg tablets**

| **Composition** | **Formula (mg)** |
|---|---|
| Compound of formula **II** | 5 |
| Mannitol | 35.275 |
| Microcrystalline cellulose | 70 |
| Croscarmellose sodium | 3.6 |
| Sodium dodecyl sulfate | 0.125 |
| Hydroxypropyl cellulose | 4.8 |
| Magnesium stearate | 1.2 |
| Purified water | Proper amount |

Procedures:
1) Mannitol, microcrystalline cellulose, and croscarmellose sodium were mixed to prepare a mixture A for further use.
   Preparation of a drug substance suspension: Hydroxypropyl cellulose was dissolved in the prescribed amount of purified water to prepare a 4% (w/w) hydroxypropyl cellulose solution; sodium dodecyl sulfate was dissolved; the compound of formula **II** was added and dispersed by stirring to prepare the drug substance suspension.
2) Fluidized bed granulation and drying: The drug substance suspension was applied to the mixture A by spraying for fluidized granulation. Granulation parameters: inlet air temperature: 55-80 °C, atomizing pressure: 600-1000 mbar, material temperature: 25-35 °C. Drying started after the spraying, and ended when the material temperature was higher than 45 °C. The materials were sized in a grinding and granulate machine through a sieve with a mesh size of Φ 0.6-1.2 mm, and dried granules after sizing were obtained.
3) The dried granules after sizing and magnesium stearate were fed into a hopper blender in sequence and well mixed to give a solid pharmaceutical composition for tableting.

### Example 1 Clinical Protocol for Medium-Risk or High-Risk Myelofibrosis Following Previous Treatment with Ruxolitinib

### 1.1 Administration regimen

Administration regimen: orally administration on an empty stomach, twice daily, 15 mg each time, at an interval of least 8 h, optimally at an interval of 12 h. Four weeks constitute one administration cycle. Dose adjustments may be made when drug-related toxic reactions occur.

Drug: the compound of formula I in 5 mg tablets, the compound of formula I being shown as a stereoisomer thereof, a compound of formula II

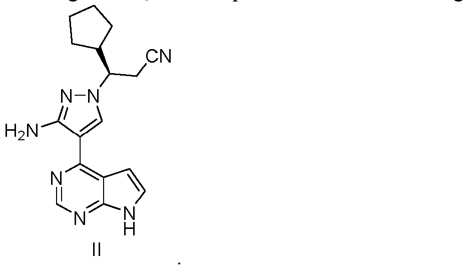

### 1.2 Enrollment criteria

1) Subjects with voluntary participation, signed informed consent, and good compliance.
2) Age: 18 years and older (when signing an informed consent); ECOG PS score: 0-2; expected survival of more than 24 weeks.
3) Those diagnosed with PMF according to WHO criteria (2016 edition), or Post-PV-MF or Post-ET-MF according to IWG-MRT criteria; being eligible for enrollment regardless of a JAK2 mutation.
4) Those with myelofibrosis assessed as medium-risk or high-risk according to DIPSS prognostic grading criteria.
5) MF subjects who has previously received treatment with ruxolitinib and are refractory/recurrent/intolerant to ruxolitinib (being refractory: a subject has been treated with ruxolitinib for a cumulative total of ≥ 3 months, and the spleen volume is reduced by < 10% or increased as detected by an MRI or CT scan compared with the spleen volume before taking ruxolitinib, or the spleen volume is reduced by < 30% or increased as assessed by palpation compared with the spleen volume before taking ruxolitinib; being recurrent: a subject has been treated with ruxolitinib for a cumulative total of ≥ 3 months, the spleen enlarges again after a response compared with the spleen volume before taking ruxolitinib, and the spleen volume is reduced by < 10% as detected by an MRI or CT scan, or is reduced by < 30% as assessed by palpation compared with the spleen volume before taking ruxolitinib; being intolerant: a subject has been treated with ruxolitinib for a cumulative total of ≥ 28 days (except for allergy), and the subject still requires red cell transfusions or has an increase in the frequency of transfusions during treatment with ruxolitinib, or experiences a reduction in platelet count of grade 3 or higher, or anemia of grade 3 or higher, or hematoma or hemorrhage of grade 3 or higher).
6) Splenomegaly: a splenic margin reaches or extends at least 5 cm below the costal margin as assessed by palpation.
7) Both peripheral blood blast cells and bone marrow blast cells in the patient are ≤ 10%.
8) Growth factors, colony stimulating factors, thrombopoietic factors, or platelet infusions are not received within 2 weeks before the examination, and hemoglobin (HGB) is ≥ 80 g/L, platelet count (PLT) is ≥ 100 × 10⁹/L, and absolute value of neutrophil count (NEUT) is ≥ 1.0 × 10⁹/L within 7 days before the first administration.
9) Major organs function normally 7 days before the first administration, that is, the indexes meet following criteria: total bilirubin (TBIL) ≤ 2× upper limit of normal (ULN); alanine aminotransferase (ALT) and aspartate aminotransferase (AST) ≤ 2.5× ULN; and serum creatinine (Cr) ≤ 1.5× ULN or creatinine clearance rate (Ccr) ≥ 50 mL/min; the results of blood coagulation function examination should meet the following criteria: prothrombin time (PT), activated partial thromboplastin time (APTT), and international normalized ratio (INR) ≤ 1.5 × ULN (not previously received anticoagulant therapy); and heart color ultrasound evaluation of left ventricular ejection fraction (LVEF) ≥ 50%.
10) Female subjects of childbearing age should agree to take necessary contraceptive measures (such as intrauterine devices, contraceptives, or condoms) during the study and for 6 months after the study; serum pregnancy test results should be negative within 7 days before the first administration, and the subjects must be in the non-lactation period; male subjects should agree to take necessary contraceptive measures during the study and for 6 months after the study.

### 1.3. Evaluation method

### 1.3.1 Evaluation of efficacy

The efficacy was evaluated by using 2013 ELN and IWG MRT consensus criteria.

Imaging assessments of the spleen volume and the efficacy of myelofibrosis were performed every 12 weeks ± 3 days, starting on day 1 of treatment cycle 1, until the subjects developed radiologically confirmed disease progression. In the evaluation of efficacy, if the palpation result of the spleen is inconsistent with the CT scan result, the CT scan result is taken as the standard.

Evaluation indexes:
the proportion of subjects with a reduction of ≥ 35% in spleen volume at the end of treatment at week 24 (SVR35);
spleen response: ① the best response rate: the proportion of subjects with at least one reduction in spleen volume ≥35% from the baseline; ② onset time: the time between the first administration and the date of the first appearance of a reduction in spleen volume of ≥ 35% from the baseline; ③ duration of maintenance of a reduction in spleen volume of ≥ 35% from the baseline (DoMSR): the time between the date of the first appearance of a reduction in spleen volume of ≥ 35% and the date of a reduction in spleen volume of < 35% from the baseline;
MF-related symptom score: ① the proportion of subjects achieved a reduction of ≥ 50% in the total symptom score of the myeloproliferative neoplasm-symptom assessment form total symptom score (MPN-SAF TSS) from the baseline at week 24; ② the reduction in the total symptom score of the MPN-SAF TSS scale from the baseline;
progression-free survival (PFS): the time between the first administration and the date of the occurrence of any one of the following events, whichever occurs first: ① a reduction in the spleen volume of ≥ 25% from the screening period; ② death of any cause;
leukemia-free survival (LFS): the time between the first administration and the date of the occurrence of any one of the following events, whichever occurs first: ① the date when the bone marrow smear shows ≥ 20% of blast cells for the first time; ② the first peripheral blood smear shows ≥ 20% of blast cells for the first time and the absolute value of blast cell count ≥ 1 × 10⁹/L for at least 2 weeks; ③ death of any cause;
overall survival (OS): the time between the first administration and death of any cause.

### 1.3.2 Evaluation of safety

The severity of adverse events was determined using the NCI CTCAE 5.0 criteria.

### 1.4 Effect data

### 1.4.1 Efficacy

The subjects experienced stable disease or partial response following treatment with the above clinical protocol. Specific data are shown in the following table:

| Subject No. | History of previous treatment | Type of gene mutation | Type of enrollment | Spleen volume during the screening period (mm³) | Spleen volume at week 12 (mm³) | Spleen volume at week 24 (mm³) |
|---|---|---|---|---|---|---|
| 1 | Ruxolitinib, danazol, thalidomide, lenalidomide, interferon, hydroxyurea, and prednisone | MPL p.W515L/K/A | Being refractory/intolerant (anemia of > grade 3 during the administration) | 523617.4 | 270692.1 | 280500.2 |
| 2 | Ruxolitinib | JAK2 V617F | Being refractory/intolerant (anemia of > grade 3 during the administration) | 3202101.3 | 2006711.7 | |

Those skilled in the art will recognize that the scope of the present application is not limited to the embodiments and examples described above. Instead, various modifications, substitutions, or recombinations may be made without departing from the spirit of the present application, all of which fall within the protection scope of the present application.

## Claims

1. A compound of formula **I,** a stereoisomer thereof, or a pharmaceutically acceptable salt for use in treating medium-risk or high-risk myelofibrosis with a previous failure of treatment with ruxolitinib:

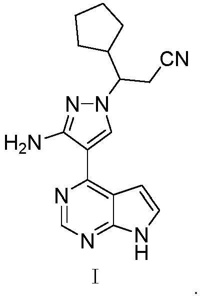

2. The compound of formula **I,** the stereoisomer thereof, or the pharmaceutically acceptable salt thereof for use according to claim 1, wherein the compound of formula **I,** the stereoisomer thereof, or the pharmaceutically acceptable salt thereof is administered to a patient in need thereof in a daily dose of 1 mg to 100 mg; preferably, the daily dose is 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or 100 mg, or a range of any two of the foregoing values as endpoints or any value therein, e.g., 1 mg to 90 mg, 5 mg to 80 mg, 10 mg to 70 mg, 15 mg to 60 mg, 20 mg to 50 mg, 20 mg to 40 mg, or 30 mg to 40 mg; further preferably, the daily dose is 1 mg to 50 mg, 5 mg to 50 mg, 5 mg to 45 mg, 5 mg to 40 mg, 10 mg to 35 mg, 10 mg to 30 mg, 20 mg to 40 mg, or 30 mg to 40 mg; further more preferably, the daily dose is 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value therein, e.g., 2 mg to 50 mg, 10 mg to 40 mg, 5 mg to 30 mg, 5 mg to 20 mg, 20 mg to 40 mg, or 30 mg to 40 mg.

3. The compound of formula **I,** the stereoisomer thereof, or the pharmaceutically acceptable salt thereof for use according to claim 1 or 2, wherein the compound of formula **I,** the stereoisomer thereof, or the pharmaceutically acceptable salt thereof is administered to the patient in need thereof once or multiple times daily; preferably, the compound of formula **I,** the stereoisomer thereof, or the pharmaceutically acceptable salt thereof is administered to the patient in need thereof once or twice daily; preferably, 28 days constitute one treatment cycle, in which the compound of formula **I,** the stereoisomer thereof, or the pharmaceutically acceptable salt thereof is administered consecutively from day 1 to day 28.

4. The compound of formula **I,** the stereoisomer thereof, or the pharmaceutically acceptable salt thereof for use according to any one of claims 1-3, wherein the compound of formula **I** is a compound of formula II:

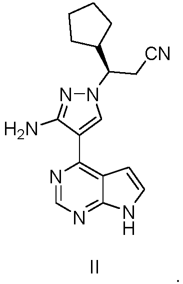

5. A pharmaceutical composition for use in treating medium-risk or high-risk myelofibrosis with a previous failure of treatment with ruxolitinib, comprising a compound of formula **I,** a stereoisomer thereof, or a pharmaceutically acceptable salt thereof.

6. The pharmaceutical composition for use according to claim 5, wherein the pharmaceutical composition is administered in twice-daily doses, with each dose being in multiple doses consisting of single doses of 5 mg, 10 mg, 15 mg, and/or 20 mg of the compound of formula **I,** the stereoisomer thereof, or the pharmaceutically acceptable salt thereof; or preferably, the pharmaceutical composition consists of single doses of 5 mg of the compound of formula **I,** the stereoisomer thereof, or the pharmaceutically acceptable salt thereof.

7. The pharmaceutical composition for use according to claim 5 or 6, wherein the pharmaceutical composition is provided in a form of a daily dose and is administered as follows: the pharmaceutical composition of the compound of formula **I,** the stereoisomer thereof, or the pharmaceutically acceptable salt thereof is administered once or twice daily; preferably, the pharmaceutical composition is administered twice daily in the same dose; further preferably, the pharmaceutical composition is administered twice daily in the same dose and at an interval of at least 8 h.

8. The compound of formula **I,** the stereoisomer thereof, or the pharmaceutically acceptable salt thereof for use according to claim 1, or the pharmaceutical composition for use according to claim 5, wherein the myelofibrosis comprises primary myelofibrosis (PMF), post-polycythemia vera myelofibrosis (Post-PV-MF), and post-essential thrombocythemia myelofibrosis (Post-ET-MF).

9. The compound of formula **I,** the stereoisomer thereof, or the pharmaceutically acceptable salt thereof for use according to claim 1, or the pharmaceutical composition for use according to claim 5, wherein the failure of treatment comprises being refractory and/or being recurrent and/or being intolerant.

10. The compound of formula **I,** the stereoisomer thereof, or the pharmaceutically acceptable salt thereof for use according to claim 1, or the pharmaceutical composition for use according to claim 5, wherein being refractory is that a patient has been treated with ruxolitinib for a cumulative total of ≥ 3 months, and the spleen volume is reduced by < 10% or increased as detected by an MRI or CT scan compared with the spleen volume before taking ruxolitinib, or the spleen volume is reduced by < 30% or increased as assessed by palpation compared with the spleen volume before taking ruxolitinib.

11. The compound of formula **I,** the stereoisomer thereof, or the pharmaceutically acceptable salt thereof for use according to claim 1, or the pharmaceutical composition for use according to claim 5, wherein being recurrent is that a patient has been treated with ruxolitinib for a cumulative total of ≥ 3 months, the spleen enlarges again after a response compared with the spleen volume before taking ruxolitinib, and the spleen volume is reduced by < 10% as detected by an MRI or CT scan, or is reduced by < 30% as assessed by palpation compared with the spleen volume before taking ruxolitinib.

12. The compound of formula **I,** a stereoisomer thereof, or a pharmaceutically acceptable salt thereof for use according to claim 1, or the pharmaceutical composition for use according to claim 5, wherein being intolerant is that a patient has been treated with ruxolitinib for a cumulative total of ≥ 28 days (except for allergy), and the patient still requires red cell transfusions or has an increase in the frequency of transfusions during treatment with ruxolitinib, or the patient experiences a reduction in platelet count of grade 3 or higher, or anemia of grade 3 or higher, or hematoma or hemorrhage of grade 3 or higher.

13. The compound of formula **I,** the stereoisomer thereof, or the pharmaceutically acceptable salt thereof for use according to claim 1, or the pharmaceutical composition for use according to claim 5, wherein the myelofibrosis is that a splenic margin of a patient reaches or extends at least 5 cm below the costal margin as assessed by palpation.

14. The compound of formula **I,** the stereoisomer thereof, or the pharmaceutically acceptable salt thereof for use according to claim 1, or the pharmaceutical composition for use according to claim 5, wherein the myelofibrosis is that both peripheral blood blast cells and bone marrow blast cells in the patient are ≤ 10%.

15. The compound of formula **I,** the stereoisomer thereof, or the pharmaceutically acceptable salt thereof for use according to claim 1, or the pharmaceutical composition for use according to claim 5, wherein the myelofibrosis comprises myelofibrosis with a mutation in a gene including but not limited to, JAK2, JAK2 V617F, MPL, CALR, ASXL1/SRSF2/IDH1/21, JAK2 exon 12, TP53, SH2B3/IDH2/U2AF1/SF3B1/EZH2/TP53, MPL p.W515L/K, CALR Type 1/Type 1-like, a triple-negative gene (no mutations in JAK2, MPL, and CALR), ASXL1, EZH2, IDH1/2, SRSF2, SF3B1, CALR/ASXL1, TP53, or U2AF1 Q157.
